# EUROPEAN PATENT SPECIFICATION

(11) **EP 2 967 329 B1**
(45) Date of publication and mention of the grant of the patent: **27.09.2023**
(21) Application number: 14709252.2
(22) Date of filing: 10.03.2014
(51) Int. Cl.: A61B 5/00

(54) **DEVICE FOR USE IN SKIN AND SCALP DIAGNOSIS, AND METHOD USING SAID DEVICE**
VORRICHTUNG ZUR VERWENDUNG IN DER DIAGNOSE VON HAUT UND KOPFHAUT, UND VERFAHREN UNTER VERWENDUNG DER VORRICHTUNG
DISPOSITIF À UTILISER DANS LE DIAGNOSTIC DE LA PEAU ET DU CUIR CHEVELU, ET PROCÉDÉ UTILISANT LEDIT DISPOSITF

(30) Priority: 11.03.2013 NL 2010416; 13.05.2013 NL 2010788
(43) Date of publication of application: 20.01.2016
(73) Proprietor: Symae Technologies Holding B.V., 5508 EG Veldhoven (NL)
(72) Inventor: ARKESTEIJN, Walter, 5508 EG Veldhoven (NL)
(74) Representative: Algemeen Octrooi- en Merkenbureau B.V.
(86) International application number: PCT/EP2014/054555
(87) International publication number: WO 2014/139934

(56) References cited:
- EP-A1- 1 736 098
- WO-A1-2012/104784
- WO-A2-2014/007869
- JP-A- 2009 245 393
- US-A1- 2003 067 545
- US-A1- 2003 076 281
- US-A1- 2004 257 439
- US-A1- 2006 109 342
- US-A1- 2009 137 908
- US-A1- 2012 307 081
- HSIN-YI TSAI ET AL: "Fluorescence image excited by a scanning UV-LED light", OPTICAL REVIEW, vol. 20, no. 2, 1 March 2013 (2013-03-01), pages 198-201, XP055089459, ISSN: 1340-6000, DOI: 10.1007/s10043-013-0034-1

## Description

### Technical field

The present disclosure relates to skin and scalp diagnosis tools, more in particular to a device which function could be considered to be an improvement over the classical Wood's lamp.

The invention relates to a device for use in diagnosing skin and/or scalp conditions. The invention further relates to a method using such a device.

### Background Art

US 2003/0076281 A1 discloses a LED system for providing diffuse illumination to obtain a substantially uniform illumination to a surface.

US 2004/0257439 A1 discloses a handheld skin observing apparatus capable of observing a skin of a person.

US 2012/0307081 A1 discloses a system and method for a hyperspectral illuminator. The hyperspectral illuminator includes an LED array for generating light of a predefined spectra.

Hsin-Yi Rsai et al.: "Fluorescence Image Excited by a Scanning UV-LED light", Optical Review, Vol. 20, no. 2, 1 March 2013 (2013-03-01) pages 198-201 discloses an optical scanning system using UV-LED light to induce fluorescence technology.

WO 2014/007869 A2 discloses a hyperspectral/multispectral imager. WO 2014/007869 A2 is a prior right document, i.e. this document is not relevant for the assessment of inventive step of the invention specified in this document.

US 2003/067545 A1 discloses a device and a method for acquiring an image of a portion of the human body.

JP2009245393 discloses a face imaging device for use in imaging a subject's face with a housing formed with an spherical space. The device comprises light sources for applying light to the inside of the space and an imaging means for imaging the portion of the entire face to which the light from the light sources is applied.

A Wood's lamp as devised in 1903, named after its inventor Robert William Wood is a diagnostic tool commonly used in dermatology, cosmetology and forensic sciences. A Wood's lamp radiates ultraviolet rays at a wavelength in spectrum between 320-400 nm and typically with the maximum emission at approximately 365 nm. In context of this disclosure; light or illumination may refer to either visible light or invisible light such as UV radiation. The Wood's lamp was first used in dermatology practice for the detection of fungal infection of hair in USA in 1925 by Margarot and Deveze.

Today, the utility of the Wood's light in a modern practice is well established. The use of it occurs predominantly in diagnostic procedures involving superficial cutaneous infections, pigmentary disorders, porphyries, malignancies, injuries and various cosmetic conditions. Wood's light is useful in diagnosing those conditions, in distinguishing them from other conditions and in locating the precise boundaries of them.

Wood's lamps are commercially available under various commercial names like Skin scanner or Skin scope. Typically, the UV sources of a very common type of Skin scopes are constructed using four fluorescent 'blacklight' tubes of the F10T8 BLB type and two tubes of the F6T5 BLB type. The acronym BLB stands for Black Light Blue; meaning that a blue filter is present suppressing much of the visible light components. In this document the term Blacklight shall refer to this particular lamp type and not the BL type which does not have this blue filter. Many lamp manufacturers produce the BLB lamp type; for example Sanyo Denki from Japan. Skin scopes using this type of light tubes are available from a wide variety of manufacturers. Also handheld Wood's lamps are available which are based upon the same technical concept using bulbs of a different shape.

Those types of Wood's lamps have many disadvantages. For instance during transport of the device the fragile glass fluorescent tubes break easily; releasing toxic substances like Mercury. Also such devices are relatively large en bulky because devices based upon BLB fluorescent tubes require heavy parts like ballasts, metal reflectors, starters and mechanical structural components.

Also, the tubes of such Wood's lamp need a few minutes warm up time before the device can be used effectively. Additionally, as such devices are switched on frequently for short periods of time; the tubes need frequent replacing because of the wear induced by those frequent starts. Another disadvantage is that the electrical components of such devices are not energy efficient and therefor produce heat of which a lot remains within the confinements of the device contributing to the discomfort of the person (also referred to as the patient) residing in the device.

The BLB fluorescent tubes produce UV on the 365 nm Mercury i-line and there is typically one phosphor present, usually consisting of europium-doped strontium fluoroborate, europium-doped strontium borate or a lead-activated calcium metasilicate which is contained in the envelope of the Wood's glass tube.

Next to the invisible 365 nm UV the phosphor in the BLB tubes produce visible by-products, predominantly in the 405 nm range. Though, some of those visible light components are considered useful in certain diagnostic applications, it also diminishes the visibility contrast when trying to observe very faint fluorescences.

Also as these visible light components appear relatively strong compared to the fluorescence produced by the patient's skin or substances and flora present on the skin, the photographic capturing of the observations is often difficult. Mainly because modern photographic and smart phones cameras have problems with the auto-focus, gain and white balance controls in the predominantly dark-blue illumination. Also as the CMOS and CCD sensors spectral sensitivity differs significantly from that of the human eye; the captured images do not well reveal the observations as seen by humans.

Another disadvantage of the prior art is that the doctor, investigator or dermatologist (also referred to as the expert) cannot easily compare observations as seen in the Wood's light with observation as seen in daylight as this requires that the patient would get in-and-out the device for each comparison; a difficult task considering the fact that the eyes of the expert require time to adjust to large difference of luminosity in the visor and daylight.

Another drawback of such conventional devices is that in general they emit UV radiation in the range of 0,5 to 1 mW/cm². Of this energy, a high portion radiates in 325 to 350 nm range and 375 to 390 nm range; wavebands which are of little or even adverse value for many applications. Limiting the amount of UV radiation is important; as long term and high levels of UV radiation exposure are considered hazardous to the human skin and eyes.

Another drawback of the prior art is the inability to observe specific vascular skin lesions or analysis of skin surface texture, such as altered keratinization and wrinkling.

For this purpose digital imaging devices like the Canfield Visia complexion analyzer are available but as such concepts require still photography they do not allow for 'live' viewing of the skin conditions in a mirror or 'live' via video camera capture.

### Disclosure of Invention

The invention relates to a device for use in diagnosing skin and/or scalp conditions as claimed in claim 1. Further, the invention relates to a method as claimed in claim 11.

The disclosure presented here could be considered as a significant improvement in the dermatology and forensic field using Wood's light devices like the skin scope or skin scanner. Compared to these devices, the here presented device is robust, low-weight, compact, easy to transport, energy-saving and has a long life time, is free of Mercury substances, emits less harmful UV radiation and creates less heat.

Additional aspects are an improved contrast of fluorescence observations resulting from the UV radiation in respect to the visible light projections coming from the emitters. Another aspect is the ability to control major components of the Wood's light spectrum and in this way provide the expert with an additional diagnostic control parameter during the observation of an skin abnormality or condition.

Other improvements are the ability to switch between daylight, cross-parallel, parallel-parallel illumination and observation modes or the ability to gradually change the spectral composition of illumination; making it possible to instantly compare skin lesions or skin conditions under different kinds of illumination.

These improvements also allow the capturing of photographs and digital images which are of improved quality and higher contrast.

The here disclosed device also can be put in an illumination mode which mimics the spectral emission of a classical Wood's lamp and in this way recreate the illumination conditions with which dermatologists and cosmetologists have been trained with in the past.

Reflectors included in the illumination units of the device for use in diagnosing skin and/or scalp conditions are provided for directing the emitted rays towards the surface under observation with a wide beam and in this way minimise the formation of shadows and shading patterns.

Exemplary embodiments of a skin diagnosis device may further comprise:
- Emitters which are constructed as:
   a. A carrier on which, in addition to UV radiating LEDs, at least one or multiple:
   b. monochromatic LEDs;
   c. white LEDs are mounted, and
- A visor; allowing the diagnostic expert to observe the patient inside the confinements of the device;
- A patient mirror; allowing the patient to see their skin conditions with their own eyes.
- An additional illumination and observation system, comprising:
   1. A controllable horizontally polarised light source directly illuminating the target area;
   2. A controllable vertically polarised light source directly illuminating the target area;
   3.A horizontal polarisation filter in front of the expert observation window;
   4.A horizontal polarisation filter in front of the patient mirror.
- An illumination control system; allowing the switching and intensity control of any of the individual illumination sources and the automatic termination of the device to keep radiation exposure well within the limits.
- A user display; showing the status and setting of the current illumination mode.

In one aspect, the present disclosure concerns a device for observing scalp and skin conditions, comprising a visor 3 to which the expert 1 can observe the patient 2 which head is placed in front of the device enclosure 6. The device can be placed on a table using the base mount 7. The expert 1 can manipulate the functions of the device using the controls 4.

On the inside a filter window 9 is placed blocking UV rays straying off towards the expert. The patient 2 can observe his/her skin in the mirror 8 during a consultation.

A light shield 10 is used to block off environmental light. Such light proof fabric could be mounted on a spiral 11 like construction for easy folding and compact transportation of the light shade. Overlapping curtain flaps 12 prevent light from entering the confinements of the device.

On the inside of the housing; emitters 14 are mounted in front of reflecting surfaces 13. The shape of these reflectors is constructed in such a way that the combination of an emitter and reflector form an illumination geometry resulting in a wide beam of rays 16 projecting on the patient's 2 face 15.

The device can be equipped with multiple of such illumination geometries; allowing the projection of overlapping rays from different angles on the face 15 resulting in a homogenous shadow and 'hot-spot' free illumination of the whole face 15. The emitter 14 can exist out of one 19 or multiple 18,20 light sources. These 18,19,20 light sources are closely mounted together on a carrier 17 which has a specific orientation relative to the reflector 13 with the result that the combination of it with the shape of the reflector creates an illumination beam illuminating the face.

For example but not limited to, a first light source 19 can be a white LED; creating a light beam 22, a second light source 20 can be an UV LED with a peak wavelength of 365 nm; creating a light beam 23 and a third 18 light source can be a LED having a peak wavelength of 405 nm; creating a light beam 21.

Depending on the application, light sources can be added which emission spectrum is considered to be of relevancy in the forensic science or dermatology field. Examples of useful applications of different illumination spectra can for example be found in the *Handbook of Biomedical Fluorescence* by Mary-Ann Mycek and Brian W. Pogue, 2003.

In figure 9a,b,c it is shown that the light beam 22,23,21 projections are very similar for each of the light sources 19,20,18 thus allowing the rapid switching of illumination modes while the way of object illumination is very similar. This consistency in illumination is considered to be very importance in the field of dermatology as small variations in the appearance of skin lesions can easily be mistaken as illumination artefacts.

Figure 9d shows a very particular feature of the disclosure. The here disclosed construction allows the blending of multiple light sources radiating at various intensities while maintaining a very similar object illumination pattern. This feature will be further detailed in this disclosure.

Figure 10 shows the spectral emission of so called 'blacklights' of the 'BLB type'. Fluorescent tubes as used in many the prior art Wood's lamps, of which examples are shown in figure 1, are of this type.

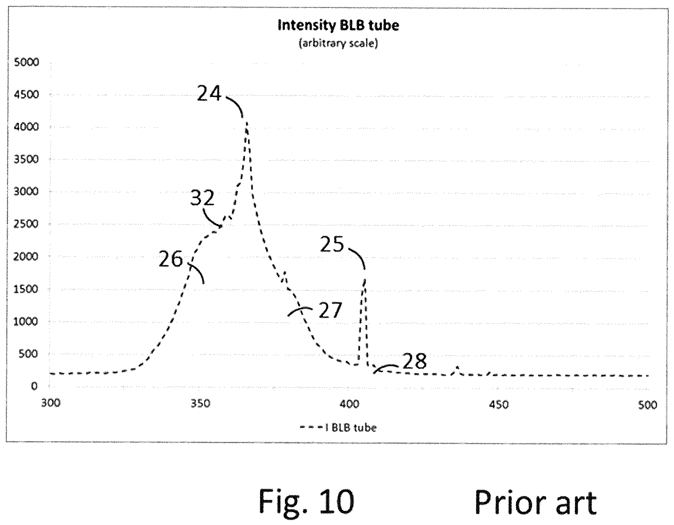

In figure 10 the typical spectral emission is shown of a common type of BLB Wood's light tube. It shows the typical Mercury emissions, in particular the i-line at 365 nm and the h-line at 404,5 nm. The phosphor in the envelope creates the emissions bands 26 and 27 which are surrounding the i-line peak emission.

In forensic sciences and dermatology both the 365 nm and 405 nm areas, collectively called the Wood's spectrum, are of interest either for observing the subsequent fluorescence from the skin or the direct observation of skin conditions lighting up in the visible blue light components illuminating the skin.

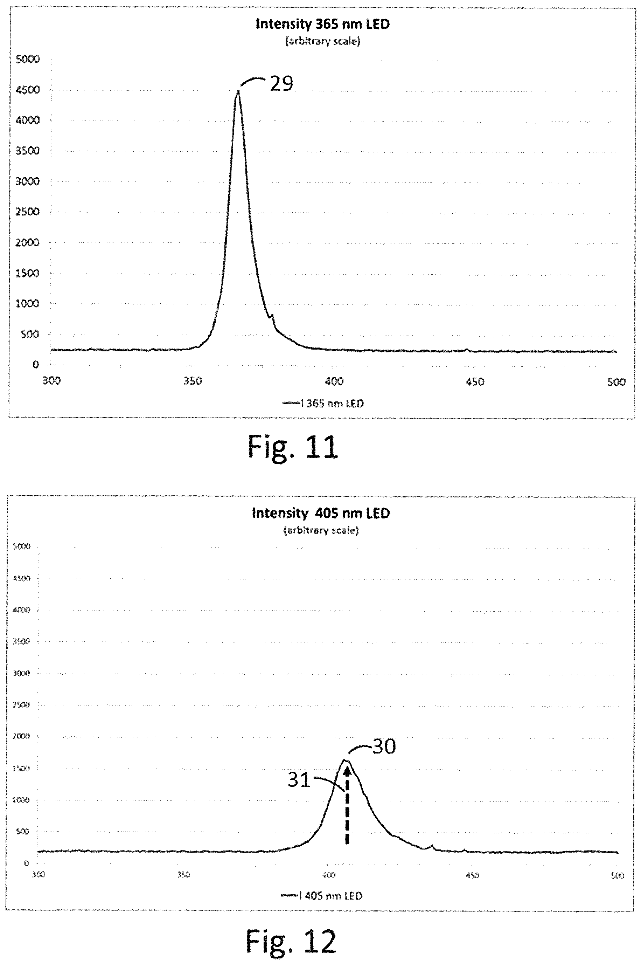

The disclosure presented here makes us of solid states illumination and radiation sources like LEDs or laser diodes which in this context are collective referred to as LEDs. Figure 11 shows the spectral emission 29 of a narrow band 365 nm LED 20 which is widely commercially available. The emission intensity peak of this light source 20 is exactly on the Mercury i-line.

Figure 12 shows the spectral emission 30 of a typically commercially available 405 nm LED. The emission peak of such a LED is on the Mercury h-line. LEDs have the advantage over fluorescence tubes that the radiation intensity 31 can be regulated easily using common techniques like current drive or pulse-width modulation.

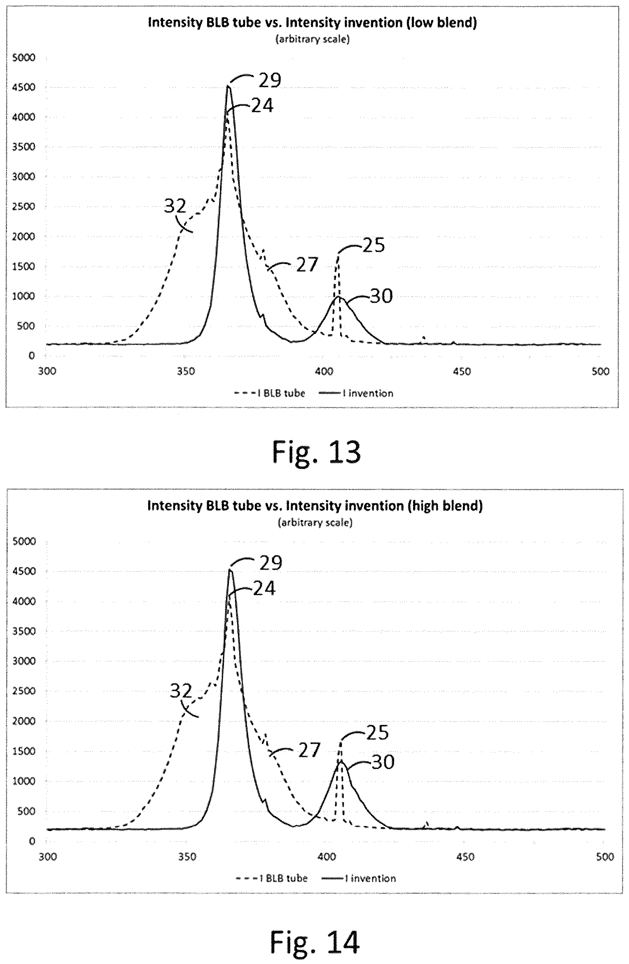

In figure 13 the emission spectrum of a typical BLB tube (dotted) is plotted against the emission spectrum of the emitter 14 in which the 405 nm source 18 and the 365 nm source 20 have been driven in such a way that to the human eye the illumination looks appears similar to that of a conventional Wood's light. In the context of this disclosure this is also referred to as 'simulated Wood's light.'

This particular LED source 20 also doesn't have the sidebands 27 and 32 which are intrinsically produced by-products of the BLB type tube. In this way, the disclosure does not expose the skin and eyes to excessive radiation energy.

The disclosed device allows the graduate control of the emission intensities of each of the individual light sources 18,19,20. It is particularly interesting to regulate light sources 18 and 20 and in this way control the blend of the Wood's light spectrum components. In figure 13 and 14 it is demonstrated how an intensity change of source 18 affects the spectrum generated by the emitter 14.

In this way the expert can study the effect of such spectral blend change on the appearance of skin lesions reacting differently from healthy skin to the exposure these different illumination blends.

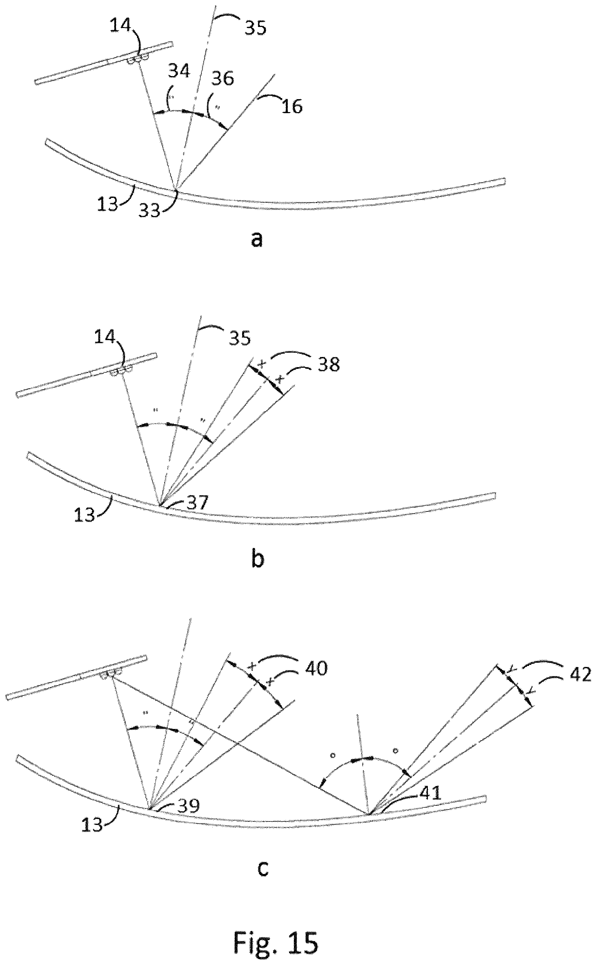

Figure 15a shows a situation where the reflector 13 has a mirror-like surface finish. The light coming from the emitter reflects 33 specular on the surface 33 towards 16 the object being illuminated. The law of reflection is applicable; meaning that the angle of incidence 34 in relationship with the normal line equals the angle of reflection 36.

Though this kind of specular reflection is efficient in projecting the light effectively on the target area, it has two drawbacks in this application. The first is that any optical impurities or irregularities in the optics of the emitter or irregularities on the mirror-like surface finish of the reflector will lead to projection artefacts like hot-spots, casted shadows or optical projection patterns.

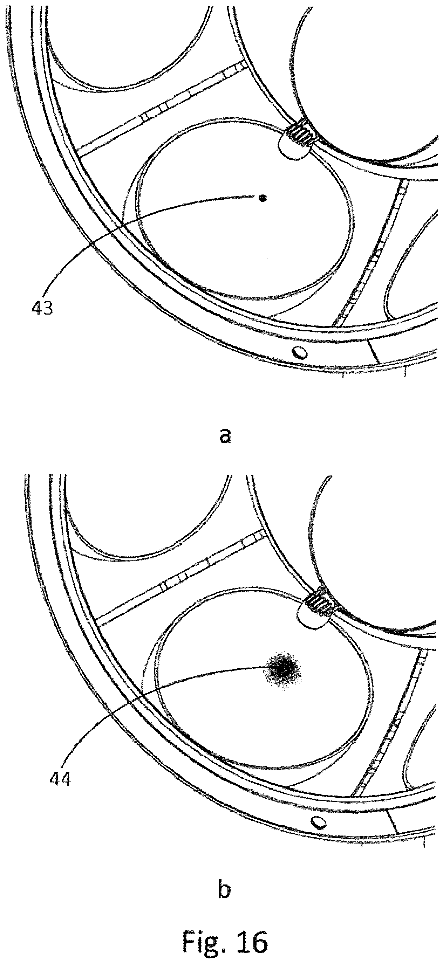

The second drawback is ocular safety. A mirror-like 37 finish will allow the bright light coming from the emitter to reflect in a specular way into the eye of the patient 2 as illustrated in figure 16a. Effectively, the eyes of the patient are directly exposed to the UV radiation and light coming from the light sources 18,19 and 20.

Guidelines for UV and blue light exposure limits and threshold values are published by the American Conference of Governmental Industrial Hygienists and the European Parliament and the Council. A particular relevant publication is the 2006/25/EC directive.

One of the most important challenges was to devise a way to expose the skin to UV radiation which is sufficient to make clear diagnostic observations while also respecting the ocular safety guidelines.

Effectively, an important challenge during the development was to create a way in which the 'apparent source size' becomes large enough to keep the exposure of the eye from all viewing conditions within these safety limits while also maintaining reflector efficiency. The solution was found in the application of a light diffusing element 37. Examples of such diffusing elements are a matted surface or a facetted surface pattern.

Figure 15b shows how the rays diffusely reflect 39 on the surface of the reflector 13 and radiate away from the mirror in a various angles 38.

As illustrated in figure 16, the 'apparent source size' becomes larger 44 which effectively reduces the exposure of the eyes to hazardous radiation.

However, increasing the diffusing capacity of the surface of the reflector leads to a reduction in directional reflection efficiency of the reflector especially when the angle of incidence increases. A solution was devised by means of adapting the diffusing capacity of the reflector 13 surface depending on the area.

Figure 15c shows that reflected 39 light with a small angle of incidence can have a smaller spread 40 angle then light reflecting 41 at a large angle of incidence. This is accomplished by applying a high diffusing texture on areas 39 with a low angle if incidence. This diffusing texture gradually becomes finer at higher angles of incidence; hence the narrow angled emission beam 42.

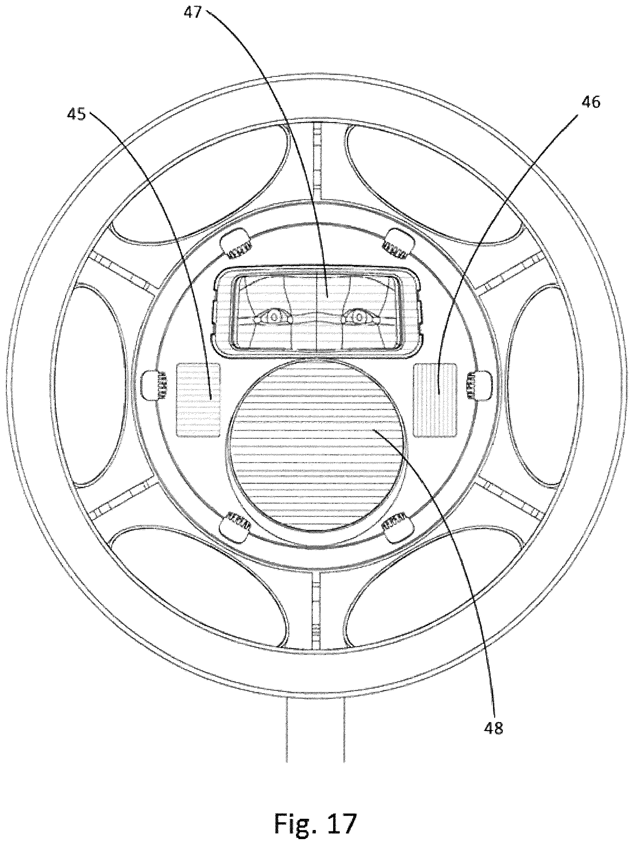

In another exemplary embodiment, of which an example embodiment is illustrated in figure 17 the device can additionally be equipped with so called parallel illumination 45 light sources and cross polarisation 46 light sources in combination with linear polarisation filters in front of the observer 47 and in front of the patient mirror 48.

In this way the expert 1 can also switch to direct polarised light illumination and observe in the cross-polarised mode for example the observation of vascular skin conditions not very well seen in other modes and skin surface textures; conditions like fine wrinkles or keratinization in the parallel polarised mode.

The size and shape of the example embodiments as disclosed are suitable for the observation of objects having roughly the size of the human head. However, the here disclosed concept is scalable in size and shape. E.g. an alternative of the disclosure (not illustrated) could be made smaller so it can be used on smaller skin areas; for instance on a portion of the face or body. For instance an example design could have the shape of a reversed bucket covering a whole side of the face from the jaw, cheek, nose and under-eye area.

In the current disclosure the expert 1 can view the patient 2 via the viewing port 3 in the device housing. In a variation of this embodiment a holder for a camera, smartphone or tablet can be placed on the location of the viewing port 1. In this way the images can be viewed and stored in a digital manner.

### Brief Description of Drawings

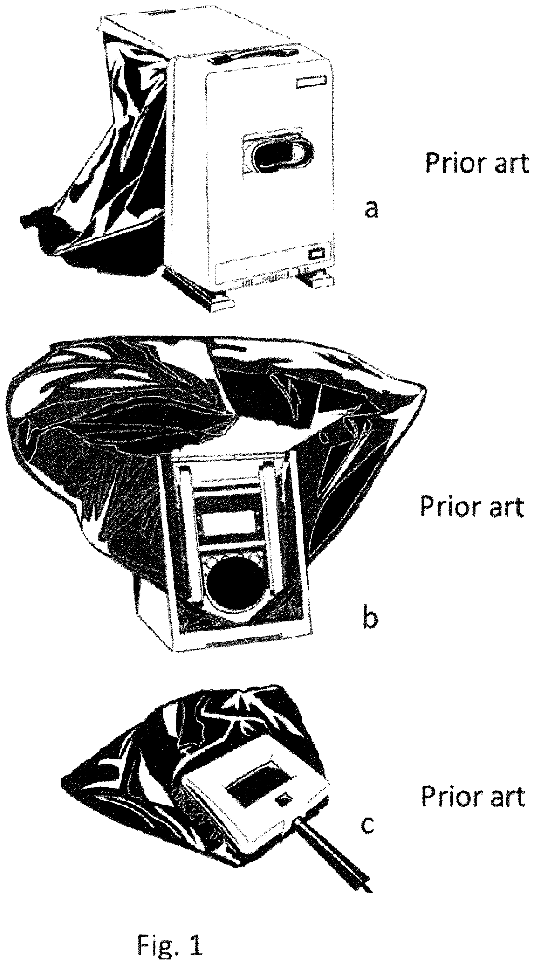

With the exception of figures 1a,b,c, the accompanying drawings which are incorporated in and form a part of the specification, illustrate the embodiments of the present disclosure and together with the written description serve to explain the principles, characteristics, and features of the disclosure. In the drawings:
- Figure 1a,b,c show images of prior art devices.

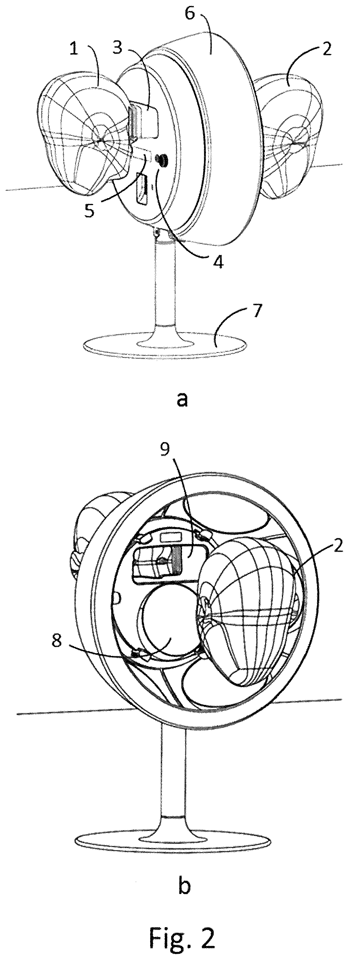

- Figure 2a,b are perspective side views of the head of the patient, the head of the expert and an exemplary embodiment of the device without mounted light shield.

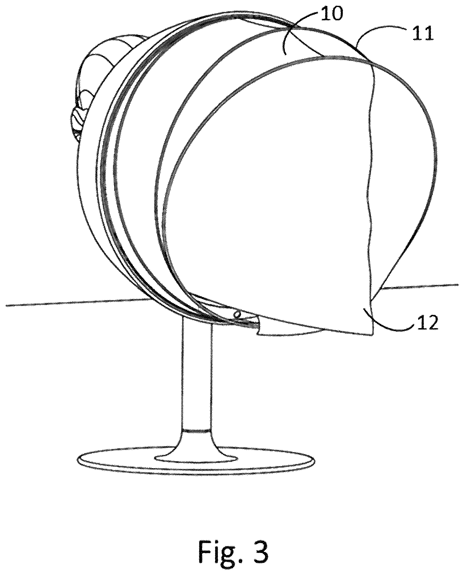

- Figure 3 shows the perspective side view of the exemplary embodiment of the device showing the device with light shield.

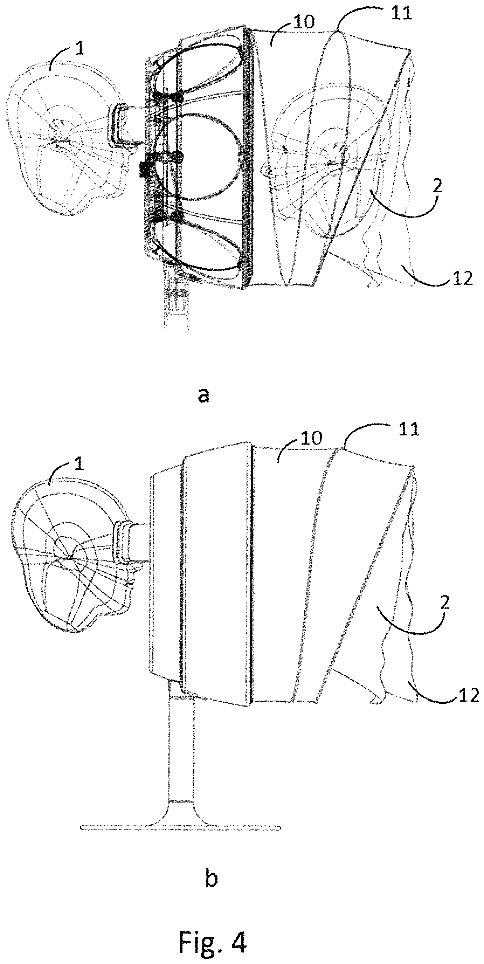

- Figure 4a,b show views from a side of the device.

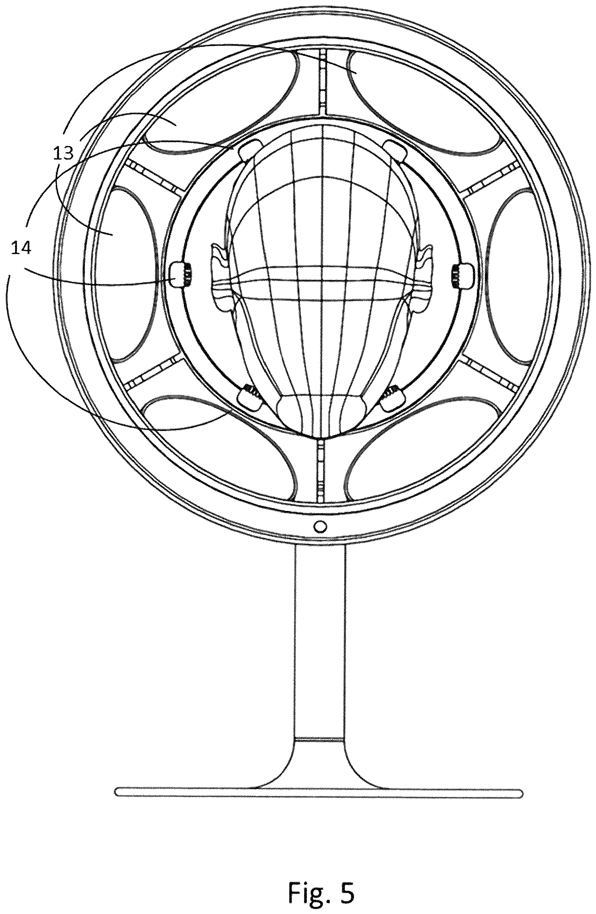

- Figure 5 shows the frontal view of the exemplary embodiment of the device showing the device without light shield.

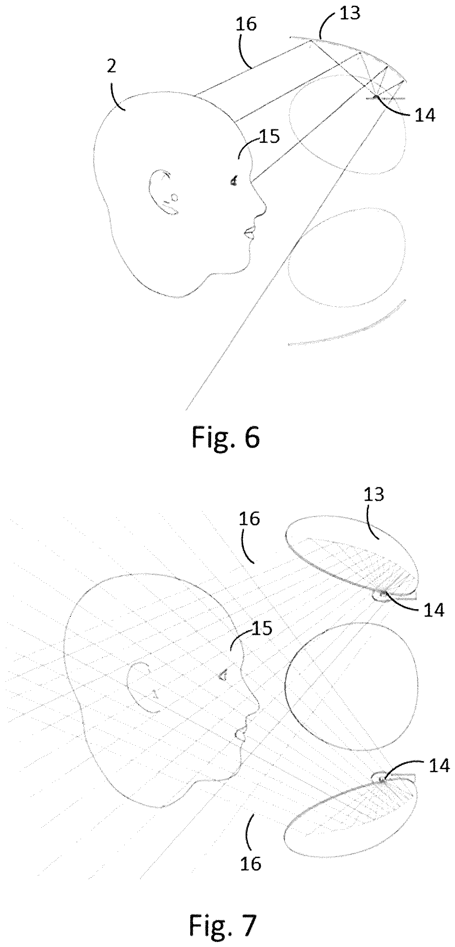

- Figure 6 shows a diagrammatic view of the light projection of one illumination geometry.
- Figure 7 shows a diagrammatic view of the light projection of multiple illumination geometries.

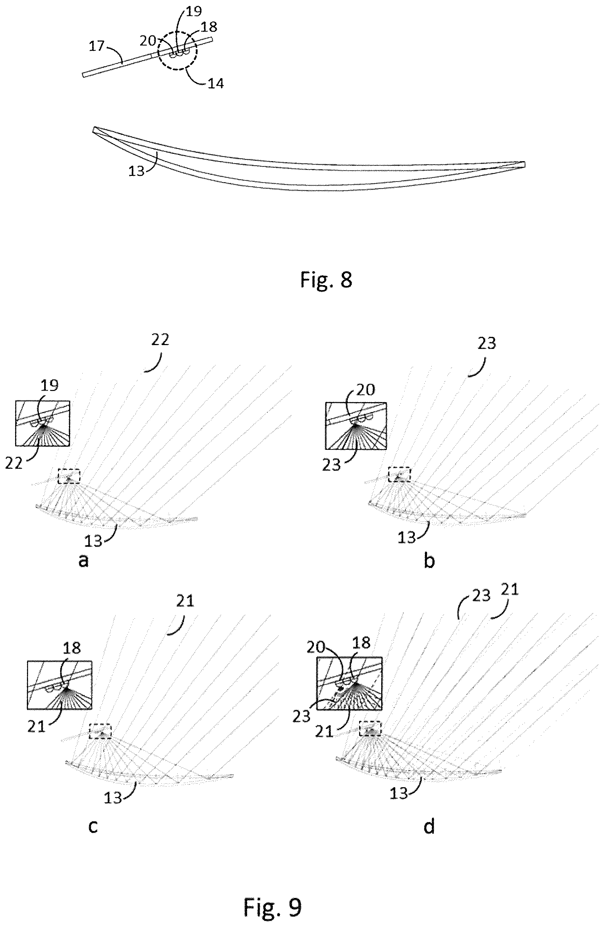

- Figure 8 shows a diagrammatic view of an exemplary illumination geometry.
- Figure 9a,b,c,d, show the projection rays of the light sources.
- Figure 10 shows a spectral emission graph of a prior art BLB type fluorescent tube.
- Figure 11 shows a spectral emission graph of a 365 nm UV LED.
- Figure 12 shows a spectral emission graph of a 405 nm UV LED.
- Figure 13 and 14 show the spectral emissions of a prior art BLB tube versus the spectral emissions of emitters incorporated in the device.
- Figure 15a,b,c are diagrammatic views of an exemplary embodiments of the illumination geometry.
- Figure 16a,b illustrate the effect of the surface diffusing surface finishes as illustrated in figure 15a and b on the 'apparent source size'.
- Figure 17 illustrate exemplary embodiments in which a cross- and parallel polarisation lights and observation windows have been added.

## Claims

1. Device for use in diagnosing skin and/or scalp conditions comprising a viewing port (3), illumination units, wherein each illumination unit comprises
- an emitter (14) for radiating a light beam comprising a carrier (17) on which UV radiating LEDs (18, 20) are mounted; and
- an optical member comprising a reflector (13) for directing and widening the light beam radiated from the emitter (14),
wherein each reflector (13) of the illumination units comprises a light diffusing element (37) for diffusing the light beam, wherein the light diffusing element (37) comprises a structure spread over a surface of the optical member to direct the emitted rays towards the surface under observation with a wide beam and in this way minimize the formation of shadows and shading patterns, wherein the device further comprises a light shield (10, 11, 12) to block off environmental light such that diagnosing can be based on observations as a result of the light propagating in the beam.

2. Device according to claim 1, wherein the optical member comprises a lens.

3. Device according to claim 1 or 2, wherein a holder for a camera, smartphone or tablet can be placed on the location of the viewing port .

4. Device according to claim 3, wherein the structure varies over the surface dependent on the angle of incidence of the light propagating in the beam with respect to the surface.

5. Device according to any one of the preceding claims, wherein the emitter (14) comprises multiple corresponding light sources and/or multiple different light sources.

6. Device according to any one of the preceding claims, wherein the UV LEDs (18, 20) have a peak wavelength of 365 nm, and/or the emitter further comprises an LED having a peak wavelength of 405 nm, and/or the emitter (14) further comprises a white LED (19) able to radiate white light.

7. Device according to any one of the preceding claims, wherein the illumination units can be independently turned on or off and/or can be independently varied in intensity, preferably the light sources can be controlled to simulate the Wood' s light spectrum components.

8. Device according to any one of the preceding claims, wherein the device comprises a visor (3), preferably the visor comprises a polarisation filter.

9. Device according to any one of the preceding claims, wherein the device comprises a patient mirror (8), preferably the patient mirror comprises a polarisation filter.

10. Device according to claim 8 in combination with a camera which is positioned or can be positioned near the visor (3) such that by means of the camera images seen through the visor can be stored.

11. Method using a device according to any one of the preceding claims, and comprising the following steps:
- radiating a light beam by emitters (14); and
- directing and widening the light beam by reflectors (13).

12. Method according to claim 11, wherein the light beam propagates through a lens before directing and widening the light beam.

13. Method according to claim 11 or 12, wherein radiating a light beam is a result of turning on multiple corresponding and/or multiple different light sources (18, 19, 20), and/or the light has a peak wavelength of 365 nm and/or a peak wavelength of 405 nm and/or the light contains white light.

14. Method according to any one of the preceding claims 11-13, wherein the light sources (18, 19, 20) can be independently turned on or off and/or can be independently varied in intensity, preferably the light sources can be controlled to simulate the Wood' s light spectrum components.

15. Method according to any one of the preceding claims 11-14, wherein the method also comprises the step of observing through a visor (3) the skin exposed to the light of the device.

## Patentansprüche

1. Vorrichtung zur Verwendung beim Diagnostizieren von Haut- und/oder Kopfhauterkrankungen, umfassend ein Beobachtungsfenster (3), Beleuchtungseinheiten, wobei jede Beleuchtungseinheit umfasst:
einen Emitter (14) zum Ausstrahlen eines Lichtstrahls, der einen Träger (17) umfasst, auf dem UV-strahlende LEDs (18, 20) montiert sind; und
ein optisches Element mit einem Reflektor (13) zum Leiten und Aufweiten des vom Emitter (14) ausgestrahlten Lichtstrahls,
wobei jeder Reflektor (13) der Beleuchtungseinheiten ein Licht diffundierendes Element (37) zum Diffundieren des Lichtstrahls umfasst, wobei das Licht diffundierende Element (37) eine Struktur umfasst, die über eine Oberfläche des optischen Elements verteilt ist, um die emittierten Strahlen zur Oberfläche unter Beobachtung mit einem breiten Strahl zu leiten und auf diese Weise die Bildung von Schatten und Schattierungsmustern zu minimieren, wobei die Vorrichtung ferner einen Lichtschutz (10, 11, 12) zum Abblocken von Umgebungslicht umfasst, derart dass das Diagnostizieren auf Beobachtungen als Ergebnis des Lichts basiert werden kann, das sich im Strahl ausbreitet.

2. Vorrichtung nach Anspruch 1, wobei das optische Element eine Linse umfasst.

3. Vorrichtung nach Anspruch 1 oder 12, wobei ein Halter für eine Kamera, ein Smartphone oder ein Tablet an der Stelle des Beobachtungsfensters platziert werden kann.

4. Vorrichtung nach Anspruch 3, wobei die Struktur über die Oberfläche in Abhängigkeit vom Einfallswinkel des sich im Strahl ausbreitenden Lichts in Bezug auf die Oberfläche variiert.

5. Vorrichtung nach einem der vorhergehenden Ansprüche, wobei der Emitter (14) mehrere entsprechende Lichtquellen und/oder mehrere verschiedene Lichtquelle umfasst.

6. Vorrichtung nach einem der vorhergehenden Ansprüche, wobei die UV-LEDs (18, 20) eine Spitzenwellenlänge von 365 nm aufweisen und/oder der Emitter ferner eine LED mit einer Spitzenwellenlänge von 405 nm aufweist und/oder der Emitter (14) ferner eine weiße LED (19) umfasst, die zum Ausstrahlen von weißem Licht in der Lage ist.

7. Vorrichtung nach einem der vorhergehenden Ansprüche, wobei die Beleuchtungseinheiten unabhängig ein- oder ausgeschaltet werden können und/oder ihre Intensität unabhängig geändert werden kann, wobei die Lichtquellen vorzugsweise so gesteuert werden können, dass sie die Wood-Komponenten des Lichtspektrums simulieren.

8. Vorrichtung nach einem der vorhergehenden Ansprüche, wobei die Vorrichtung ein Visier (3) umfasst, wobei das Visier vorzugsweise ein Polarisationsfilter umfasst.

9. Vorrichtung nach einem der vorhergehenden Ansprüche, wobei die Vorrichtung einen Patientenspiegel (8) umfasst, wobei der Patientenspiegel vorzugsweise ein Polarisationsfilter umfasst.

10. Vorrichtung nach Anspruch 1 in Kombination mit einer Kamera, die in der Nähe des Visiers (3) positioniert ist oder positioniert werden kann, derart dass mittels der Kamera Bilder, die durch den Blendschutz gesehen werden, gespeichert werden können.

11. Verfahren zur Verwendung einer Vorrichtung nach einem der vorhergehenden Ansprüche und umfassend die folgenden Schritte:
Ausstrahlen eines Lichtstrahls durch Emitter (14); und
Leiten und Aufweiten des Lichtstrahls durch Reflektoren (13).

12. Verfahren nach Anspruch 11, wobei der Lichtstrahl sich vor dem Leiten und Aufweiten des Lichtstrahls durch eine Linse ausbreitet.

13. Verfahren nach Anspruch 11 oder 12, wobei das Ausstrahlen eines Lichtstrahls ein Ergebnis des Einschaltens mehrerer entsprechender und/oder mehrerer verschiedener Lichtquellen (18, 19, 20) ist und/oder das Licht eine Spitzenwellenlänge von 365 nm und/oder eine Spitzenwellenlänge von 405 nm aufweist und/oder das Licht weißes Licht enthält.

14. Vorrichtung nach einem der Ansprüche 11 bis 13, wobei die Lichtquellen (18, 19, 20) unabhängig ein- oder ausgeschaltet werden können und/oder ihre Intensität unabhängig geändert werden kann, wobei die Lichtquellen vorzugsweise so gesteuert werden können, dass sie die Wood-Komponenten des Lichtspektrums simulieren.

15. Verfahren nach einem der vorhergehenden Ansprüche 11 bis 14, wobei das Verfahren außerdem den Schritt des Beobachtens der dem Licht der Vorrichtung ausgesetzten Haut durch ein Visier (3) umfasst.

## Revendications

1. Dispositif pour utilisation dans le diagnostic des affections de la peau et/ou du cuir chevelu comprenant un port d'observation (3), des unités d'éclairage, dans lequel chaque unité d'éclairage comprend :
- un émetteur (14) pour rayonner un faisceau lumineux comprenant un support (17) sur lequel des diodes électroluminescentes à UV (18, 20) sont montées ; et
- un organe optique comprenant un réflecteur (13) pour orienter et élargir le faisceau lumineux émis par l'émetteur (14),
dans lequel chaque réflecteur (13) des unités d'éclairage comprend un élément diffuseur de lumière (37) pour diffuser le faisceau lumineux, dans lequel l'élément diffuseur de lumière (37) comprend une structure s'étalant sur la surface de l'organe optique pour orienter les rayons émis vers la surface observée avec un faisceau large and minimiser ainsi la formation d'ombres et de motifs d'ombrage, dans lequel le dispositif comprend en outre un pare-lumière (10, 11, 12) pour bloquer la lumière ambiante de telle sorte que le diagnostic puisse être basé sur des observations résultant de la lumière se propageant dans le faisceau.

2. Dispositif selon la revendication 1, dans lequel l'organe optique comprend une lentille.

3. Dispositif selon la revendication 1 ou 2, dans lequel un support pour caméra, téléphone intelligent ou tablette peut être placé à la place du port d'observation.

4. Dispositif selon la revendication 3, dans lequel la structure varie sur la surface en fonction de l'angle d'incidence de la lumière se propageant dans le faisceau par rapport à la surface.

5. Dispositif selon l'une quelconque des revendications précédentes, dans lequel l'émetteur (14) comprend plusieurs sources lumineuses correspondantes et/ou plusieurs sources lumineuses différentes.

6. Dispositif selon l'une quelconque des revendications précédentes, dans lequel les diodes électroluminescentes à UV (18, 20) présentent une longueur d'onde de crête de 365 nm, et/ou l'émetteur comprend en outre une diode électroluminescente présentant une longueur d'onde de crête de 405 nm, et/ou l'émetteur (14) comprend en outre une diode électroluminescente (19) capable de rayonner de la lumière blanche.

7. Dispositif selon l'une quelconque des revendications précédentes, dans lequel les unités d'éclairage peuvent être allumées ou éteintes de manière indépendante et/ou peuvent varier en intensité de manière indépendante, de préférence les sources lumineuses peuvent être commandées pour simuler les composantes du spectre lumineux de Wood.

8. Dispositif selon l'une quelconque des revendications précédentes, dans lequel le dispositif comprend une visière (3), de préférence la visière comprend un filtre de polarisation.

9. Dispositif selon l'une quelconque des revendications précédentes, dans lequel le dispositif comprend un miroir de patient (8), de préférence le miroir de patient comprend un filtre de polarisation.

10. Dispositif selon la revendication 8 en combinaison avec une caméra qui est positionnée ou peut être positionnée près d'une visière (3) de telle sorte que des images vues à travers la visière peuvent être stockées au moyen de la caméra.

11. Procédé utilisant un dispositif selon l'une quelconque des revendications précédentes, et comprenant les étapes de :
- émission d'un faisceau lumineux par des émetteurs (14) ; et
- orientation et élargissement du faisceau lumineux par des réflecteurs (13).

12. Procédé selon la revendication 11, dans lequel le faisceau lumineux se propage à travers une lentille avant d'orienter et d'élargir le faisceau lumineux.

13. Procédé selon la revendication 11 ou 12, dans lequel le faisceau lumineux résulte de l'allumage d'une pluralité de sources lumineuses (18, 19, 20) correspondantes et/ou différentes, et/ou la lumière présente une longueur d'onde de crête de 365 nm et/ou une longueur d'onde de crête de 405 nm et/ou la lumière contient de la lumière blanche.

14. Procédé selon l'une quelconque des revendications précédentes 11-13, dans lequel les sources lumineuses (18, 19, 20) peuvent être allumées ou éteintes de manière indépendante et/ou peuvent varier en intensité de manière indépendante, de préférence les sources lumineuses peuvent être commandées pour simuler les composantes du spectre lumineux de Wood.

15. Procédé selon l'une quelconque des revendications précédentes 11-14, dans lequel le procédé comprend également une étape d'observation de la peau exposée à la lumière du dispositif à travers une visière.
